## Europäisches Patentamt

## European Patent Office

## Office européen des brevets

(19)

(11) Veröffentlichungsnummer : **0 185 693 B1**

(12) # EUROPÄISCHE PATENTSCHRIFT

(45) Veröffentlichungstag der Patentschrift :
23.03.88

(21) Anmeldenummer : 85902535.5

(22) Anmeldetag : 23.05.85

(86) Internationale Anmeldenummer :
PCT/EP 85/00250

(87) Internationale Veröffentlichungsnummer :
WO/8505641 (19.12.85 Gazette 85/27)

(51) Int. Cl.$^4$ : **C 12 Q  1/58, C 12 N  9/02,
C 12 N  9/88**

(54) **VERFAHREN UND REAGENZ ZUR VOLLENZYMATISCHEN BESTIMMUNG VON HARNSTOFF.**

(30) Priorität : 25.05.84 DE 3419642

(43) Veröffentlichungstag der Anmeldung :
02.07.86 Patentblatt 86/27

(45) Bekanntmachung des Hinweises auf die Patenterteilung : 23.03.88 Patentblatt 88/12

(84) Benannte Vertragsstaaten :
AT BE CH DE FR GB IT LI LU NL SE

(56) Entgegenhaltungen :
DE-A- 3 247 894
Chemical Abstracts, vol. 66, no. 10, 6 March 1967 (Columbus, Ohio, US), F. Trijbels et al.: "Allantrate and ureidoglycolate degradation by Pseudomonas aeruginosa", abstract 43898d, page 4159
Chemical Abstracts, vol. 75, no. 13, 27 September 1971, (Columbus, Ohio, US), Van der Drift et al.: "S-Ureidoglycolate dehydrogenase. Purification and properties", abstract 84664x, page 43

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : SIEDEL, Joachim
Bahnhofstrasse 6
D-8131 Bernried (DE)
Erfinder : WAHLEFELD, August, Wilhelm
Alpenblickstrasse 25
D-8126 Hohenpeissenberg (DE)
Erfinder : ZIEGENHORN, Joachim
Ina-Seidel-Weg 1
D-8130 Starnberg (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

**0 185 693**

**Beschreibung**

Die Erfindung betrifft ein Verfahren und Reagenz zur enzymatischen Bestimmung von Harnstoff, insbesondere in Körperflüssigkeiten wie Serum, Plasma und Urin.

Innerhalb der analytischen Chemie spielt die quantitative Bestimmung von Harnstoff eine besonders wichtige Rolle in der klinisch-chemischen Diagnostik. Die Harnstoffkonzentration in Körperflüssigkeiten wie Serum oder Urin gibt dem klinischen Mediziner wichtige Aufschlüsse über die Nierenfunktion, bei Dialysepatienten darüberhinaus über den Wirkungsgrad extra- oder intrakorporaler Blutwäschen.

Gegenüber rein chemischen oder teilenzymatischen photometrischen Bestimmungsverfahren z. B. den besonders häufig eingesetzten Methoden unter Verwendung des Fearon'schen Reagenzes bzw. der Urease/Berthelot-Reaktion (zur Beschreibung s. z. B. R.J. Henry, D.C. Cannon, J.W. Winkelmann (Hrsg.), Clinical Chemistry : Principles and Techniques, 2. Ausgabe, Harper and Row, Hagerstown, Maryland, USA (1974), Seiten 503-526) gewinnen dabei vollenzymatische Analysenmethoden mehr und mehr an Bedeutung. Ihre wesentlichen Vorteile im Vergleich zu den obengenannten Verfahren sind die hohe Spezifität, ihre Störunanfälligkeit, z. B. gegenüber Medikamenteneinflüssen, Temperatur- und pH-Schwankungen, die Vermeidung des Umgangs mit korrosiven, stark sauren oder alkalischen bzw. toxischen Reagenzien, ihre universelle Einsetzbarkeit sowohl für die manuelle Testdurchführung als auch an den verschiedensten Analysenautomaten, sowie die Möglichkeit, die Meßergebnisse ohne Mitführen von Standards mit Hilfe genau definierter Umrechnungsfaktoren auswerten zu können, wobei die Bedingungen des Lambert-Beer'schen Gesetzes im Prinzip über den gesamten am Photometer ausnutzbaren Extinktionsbereich gewährleistet sind.

Bislang sind zwei verschiedene Verfahren zur vollenzymatischen Harnstoff-Bestimmung bekannt.

In einem dieser Verfahren wird Harnstoff mit Urease, E.C. 3.5.1.5., in zwei Moleküle Ammoniak und ein Molekül $CO_2$ hydrolytisch gespalten ; aus dem entstandenen Ammoniak wird in Gegenwart von alpha-Ketoglutarat, NADH und Glutamatdehydrogenase, E.C. 1.4.1.3, Glutamat gebildet. Die Abnahme der NADH-Konzentration im Reaktionsgemisch, die photometrisch bei 334, 340 oder 365 nm gemessen werden kann, ist proportional der eingesetzten Harnstoffmenge (H.U. Bergmeyer (Hrsg.), Methoden der enzymatischen Analyse, 3. Auflage, Bd. II, Verlag Chemie, Weinheim (1974), S. 1842). Nach dem zweiten Verfahren (US-PS 3, 655, 516) wird Harnstoff in einer ATP-abhängigen Reaktion mittels der Harnstoff-Amidohydrolase, E.C. 6.3.4.6, hydrolysiert, wobei pro Molekül umgesetzten Harnstoffs ein Molekül ADP entsteht. Dieses wird mit Phosphoenolpyruvat und Pyruvat-Kinase, E.C. 2.7.1.40 in ATP zurückgewandelt und das freigesetzte Pyruvat in Gegenwart von NADH und Lactat-Dehydrogenase, E.C. 1.1.1.27, zu Lactat reduziert. Meßparameter ist auch hier wieder — wie im erstgenannten Verfahren — die Abnahme der NADH-Konzentration im Reaktionsgemisch.

Von beiden Verfahren hat bis heute nur das erstere breiten Eingang in die Routineanalytik gefunden.

Trotz des deutlichen Fortschritts, den die vollenzymatische Bestimmung von Harnstoff gegenüber den rein chemischen oder teilenzymatischen Analysenmethoden mit sich brachte, weisen jedoch beide obengenannten enzymatischen Verfahren noch mehrere, weitgehend identische Nachteile auf : Einmal ist NADH in wäßriger Lösung, auch im neutralen pH-Bereich, relativ instabil, so daß die Haltbarkeit der gebrauchsfertigen Reagenzlösungen nur über einen kurzen Zeitraum, d. h. etwa 1 Tag bei Raumtemperatur-Lagerung oder 3 Tage bei Aufbewahrung bei 2 bis 8 °C gewährleistet ist. Darüberhinaus führen Testansätze nach dem Probe/Reagenzleerwert-Meßverfahren gelegentlich zu falsch überhöhten Harnstoffwerten, falls mit trübem bzw. stark im Bereich von 334 bis 365 absorbierendem Probenmaterial gearbeitet werden muß oder vergleichsweise hohe Konzentrationen von den immer in Spuren im Serum oder Urin vorkommenden Substanzen Ammoniak oder Pyruvat vorhanden sind. Für genaue Harnstoffbestimmungen ist hier also die Mitführung eines getrennten Probenleerwert-Ansatzes erforderlich (Reagenz ohne Urease bzw. Harnstoff-Amidohydrolase), was zumindest die manuelle Testdurchführung erheblich kompliziert. Zwar können derartige Störungen prinzipiell durch Anwendung kinetischer Meßtechniken ausgeschaltet werden, die mit hinreichender Präzision jedoch nur an Analysenautomaten durchführbar sind und umgekehrt die manuelle Test-Handhabung weitgehend ausschließen.

Schließlich ist in beiden Fällen aufgrund der Eigenschaften der Indikatorreaktion keine Möglichkeit gegeben, ein farbgebendes Visualisierungssystem anzukoppeln, das einerseits Störungen durch Probentrübungen bzw. -eigenfärbungen weitgehend ausschalten würde und außerdem die Verwendung von im Sichtbaren messenden Photometern erlaubt, die keine Einrichtungen für Messungen im UV-Bereich besitzen.

Es besteht daher der Bedarf an einem vollenzymatischen Verfahren und Reagenz zur spezifischen Harnstoffbestimmung, die die obengenannten Nachteile, d. H. stark eingeschränkte Lagerfähigkeit des Analysenreagenzes sowie die Miterfassung störender Probenbestandteile vermeidet und auch die Durchführung der Messung im sichtbaren Wellenlängenbereich ermöglicht.

Diese Aufgabe wird erfindungsgemäß gelöst durch ein Verfahren zur enzymatischen Harnstoffbestimmung, welches dadurch gekennzeichnet ist, daß man Harnstoff mit Glyoxylat in Gegenwart von (S)-Ureidoglycolat-Synthetase zu (S)-Ureidoglycolat umsetzt und letzteres mit $NAD^+$ oder $NADP^+$, bevorzugt mit $NAD^+$, sowie Ureidoglycolat-Dehydrogenase, E.C. 1.1.1.154, unter stöchiometrischer Bildung von NADH oder NADPH zu Carbamoyloxamat oxidiert.

2

Die Konzentrationsbestimmung von Harnstoff kann entweder direkt, d. h. über die Messung des gebildeten NADH oder NADPH zwischen 334 und 365 nm, oder bevorzugt, über ein NAD(P)H-abhängiges enzymatisches Farbindikatorsystem erfolgen.

Besonders bevorzugt wird ein System, in dem aus NADH oder NADPH in konzentrationsabhängiger Weise aus einem Tetrazoliumsalz in Gegenwart von Diaphorase, E.C. 1.6.4.3, ein Formazanfarbstoff gebildet wird.

Die dem Verfahren zugrundeliegenden Enzymreaktionen (Ureidoglycolat-Synthetase- bzw. Ureidoglycolat-Dehydrogenase-Reaktion) sind zwar seit fast 20 Jahren bekannt aus J. Bacteriology, Vol. 90, S. 1531-1536 (1965). Jedoch wird dort gelehrt, daß die Ureidoglycolatsynthese außerordentlich langsam abläuft und bei je 20 µmol Harnstoff und Glyoxylat nach 1 Stunde nur 10 % reagiert hatten. Es ist daher überraschend, daß die erfindungsgemäße Bestimmung trotzdem durchführbar ist.

Die (S)-Ureidoglycolat-Synthetase läßt sich in ausreichender Reinheit und Aktivität nach dem in J. Bacteriology 90, 1525-1530 (1965) beschriebenen Verfahren gewinnen. Das Vorkommen des Enzyms ist schon für viele Mikroorganismen beschrieben worden, wie z. B. Saccharomyces cerevisiae, Candida utilis und Streptococcen. Als Ausgangsmaterial bevorzugt wird Streptococcus allantoicus, DSM 2965.

Ein weiterer Gegenstand der Erfindung ist ein Reagenz zur Bestimmung von Harnstoff, welches dadurch gekennzeichnet ist, daß es aus einer wäßrigen, gepufferten Lösung besteht, die Glyoxylat, NAD$^+$ oder NADP$^+$, (S)-Ureidoglycolat-Synthetase und Ureidoglycolat-Dehydrogenase sowie vorzugsweise zusätzlich ein NAD(P) H abhängiges Farbindikatorsystem enthält. Besonders bevorzugt wird ein Reagenz, welches zusätzlich als NAD(P) H abhängiges Farbindikatorsystem Diaphorase und ein reduktiv in einen Formazan-Farbstoff umwandelbares Tetrazoliumsalz enthält.

Der pH-Wert der wäßrigen Lösung liegt zweckmäßig zwischen 7 und 9, vorzugsweise zwischen 7,5 und 8,5, ganz besonders bevorzugt zwischen 7,7 und 8,3. Zur Einstellung des pH-Wertes kommen alle Puffersubstanzen in Frage, deren pK-Werte zwischen pH 6,5 und 9,5 liegen. Besonders geeignet sind Phosphat- und Tris-Puffer. Die Konzentration an Puffersubstanz beträgt zweckmäßig 20 bis 200 mmol/l, bevorzugt 50 bis 150 mmol/l. Für Glyoxylat kommen Konzentrationen von 0,1 bis 10 mmol/l in Frage, bevorzugt 0,5 bis 5 mmol/l, ganz besonders bevorzugt 1 bis 3 mmol/l. Von den Coenzymen NAD$^+$ und NADP$^+$ wird NAD$^+$ bevorzugt ; die Konzentration des Coenzyms liegt günstigerweise zwischen 1 bis 20 mmol/l, vorzugsweise bei 5 bis 10 mmol/l. Für (S)-Ureidoglycolat-Synthetase sind Konzentrationen von 1 bis 30 U/ml zweckmäßig, bevorzugt 3 bis 20 U/ml, besonders bevorzugt 5 bis 10 U/ml. Gleiches gilt auch für die Ureidoglycolat-Dehydrogenase.

Die Diaphorase-Konzentration liegt normalerweise zwischen 0,1 und 10 U/ml, bevorzugt zwischen 0,5 und 5 U/ml, besonders bevorzugt zwischen 1 und 2 U/ml. Von den Tetrazolium-Salzen werden 3-(4′, 5′-Dimethylthiazol-2-yl)-2,4-diphenyltetrazoliumbromid (MTT) und 2,2′-di-(p-Nitrophenyl)-5,5′-diphenyl-3,3′-(3,3′-dimethoxy-4,4′-diphenylen)-ditetrazoliumchlorid (NBT) bevorzugt, insbesondere NBT. Beispiele für andere geeignete Tetrazoliumsalze sind INT (2-(p-iodophenyl)-3-(p-nitrophenyl)-5-phenyltetrazolium-chloride), TNBT (2,2′,5,5′-tetra-(p-nitrophenyl)-3,3′-(3-dimethoxy-4-diphenylene)-di-tetrazoliumchloride), NT (2,2′-p-diphenylene-3,3′,5,5′-tetraphenylditetrazolium chloride) und TT (2,3,5-triphenyltetrazoliumchloride). Ihre Konzentrationen im Reagenz liegen gewöhnlich zwischen 0,05 und 1 mmol/l, vorzugsweise zwischen 0,1 und 0,5 mmol/l. Um eine vorzeitige Ausfällung des gebildeten Formazans zu verhindern, wird dem Reagenz vorteilhafterweise auch ein nichtionisches Detergenz, wie z. B. Triton X-100 zugesetzt, und zwar in Konzentrationen von 0,1 bis 0,5 %, insbesondere von 0,2 bis 0,4 %.

Das folgende Beispiel erläutert die Erfindung weiter :

## Beispiel 1

Reagenz und Verfahren zur Bestimmung von Harnstoff.

A) Zur Herstellung von Ureidoglycolat-Dehydrogenase wurde Proteus rettgeri, DSM 2964, aerob in einem Medium aus 10 g Difco-Hefeextrakt, 7 g Allantoin, 0,18 g MgSO$_4$ · 7H$_2$O/1 Liter Leitungswasser aerob gezüchtet, die Biomasse abzentrifugiert und in 0,05 mol/l Tris · HCl, pH 7,0, durch Ultraschall aufgeschlossen. Zur Enzymreinigung wurde der Rohextrakt über Polyethylenimin G 35 fraktioniert und anschließend über Phenylsepharose chromatographiert, dann einer ersten Ammonsulfat-Fraktionierung, einer Chromatographie an Sepharose S-200 und schließlich einer zweiten AS-Fraktionierung unterworfen. Das gereinigte Enzym wies eine spezifische Aktivität von 144 U/mg Protein auf und wurde als Suspension in 3,2 mol/l AS, pH 8,1, mit einer Konzentration von 5 mg/ml aufbewahrt.

Vor Verwendung zur Bestimmung von Harnstoff empfiehlt es sich, das Ammoniumsulfat aus der Enzympräparation durch Dialyse gegen 0,1 mol/l Tris · HCl zu entfernen.

B) Farbreagenz

(Siehe Tabelle Seite 4 f.)

| Komponente | Konzentration im Reagenz |
|---|---|
| Tris·HCl (pH 8,0) | 100 mmol/l |
| Triton X-100 | 0,3 % |
| Na-Glyoxylat | 3 mmol/l |
| NAD$^+$ | 5 mmol/l |

| | |
|---|---|
| NBT | 0,2 mmol/l |
| (S)-Ureidoglycolat-Synthetase | 10 U/ml |
| Ureidoglycolat-Dehydrogenase | 7 U/ml |
| Diaphorase | 1 U/ml |

C) Testdurchführung

Wellenlänge 546 nm, T = 25 °C, Schichtdicke 10 mm. Messung gegen Reagenz-Leerwert.
In Küvetten pipettieren:

| | Probenwert (P) | Reagenzleerwert* (RL) |
|---|---|---|
| Farbreagenz 1.1 | 1,00 ml | 1,00 ml |
| Probe** | 0,01 ml | - |
| mischen, 20 Minuten bei 25°C inkubieren, dann innerhalb weiterer 30 Minuten $E_P$ gegen $E_{RL}$ messen ($\Delta E$). | | |

\* Pro Meßreihe genügt der Ansatz eines Reagenzleerwertes.
\*\* wäßrige Lösungen von Harnstoff mit Konzentrationen von 0,5 bis 5 nmol/l.

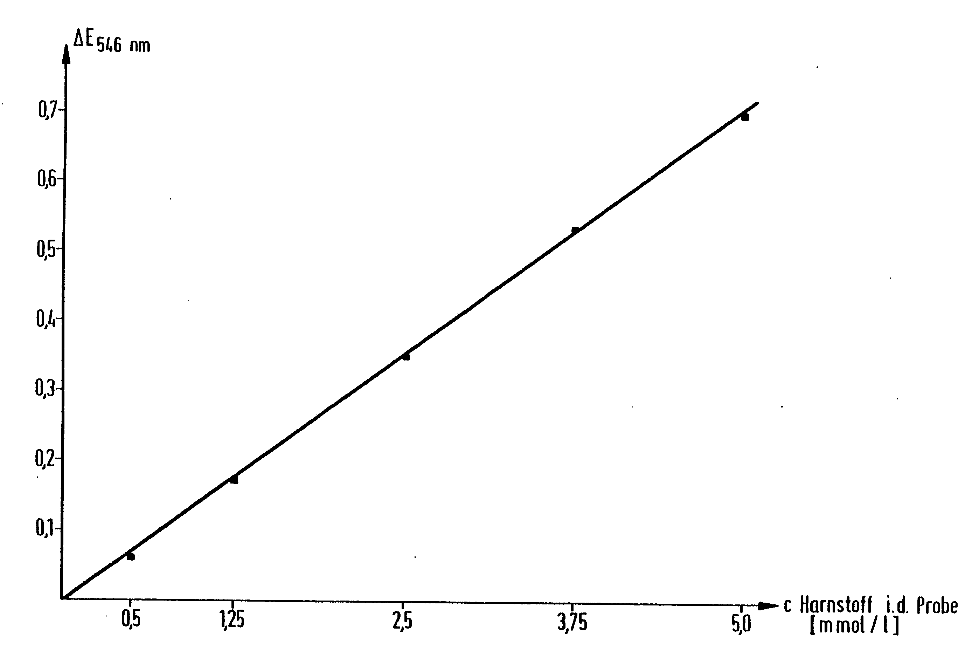

Die Abhängigkeit von ΔE gegen die Konzentration an Harnstoff in der eingesetzten Probe ist in Fig. 1 der beigefügten Zeichnung aufgetragen.

**Patentansprüche**

1. Verfahren zur enzymatischen Bestimmung von Harnstoff, dadurch gekennzeichnet, daß man den Harnstoff mit Glyoxylat in Gegenwart von (S)-Ureidoglycolat-Synthetase zu (S)-Ureidoglycolat umsetzt und letzteres mit NAD(P)$^+$ und Ureidoglycolatdehydrogenase zu Carbamoyloxamat oxidiert und gebildetes NAD(P)H entweder direkt oder über ein Farbindikatorsystem mißt.

2. Verfahren nach Anspruch 1, dadurch gekennzeichnet, daß man NAD(P)H mit einem Tetrazoliumsalz in Gegenwart von Diaphorase zu einem Formazanfarbstoff umsetzt und letzteren mißt.

3. Verfahren nach Anspruch 1 oder 2, dadurch gekennzeichnet, daß man Ureidoglycolat-Dehydrogenase aus Proteus rettgeri, DSM 2964 verwendet.

4. Reagenz zur enzymatischen Bestimmung von Harnstoff, dadurch gekennzeichnet, daß es Glyoxylat, NAD(P)$^+$, (S)-Ureidoglycolat-Synthetase, Ureidoglycolat-Dehydrogenase und Puffersubstanz enthält.

5. Reagenz nach Anspruch 4, dadurch gekennzeichnet, daß es zusätzlich Diaphorase und Tetrazoliumsalz enthält.

6. Reagenz nach Anspruch 5, dadurch gekennzeichnet, daß es in Form der wäßrigen Lösungen 0,1 bis 10 mmol/l Glyoxylat, 1 bis 20 mmol/l NAD(P)$^+$, je 1 bis 30 U/ml (S)-Ureidoglycolat-Synthetase und Ureidoglycolat-Dehydrogenase, 0,1 bis 10 U/ml Diaphorase, 0,05 bis 1 mmol/l Tetrazoliumzalz und 20 bis 200 mmol/l Puffersubstanz pH 7 bis 9 enthält.

7. Reagenz nach Anspruch 6, dadurch gekennzeichnet, daß es 0,5 bis 5 mmol/l Glyoxylat, 5 bis 10 mmol/l NAD$^+$, je 3 bis 30 U/ml (S)-Ureidoglykolat-Synthetase und Ureidoglykolat-Dehydrogenase, 0,5 bis 5 U/ml Diaphorase und 0,1 bis 0,5 mmol/l Tetrazoliumsalz und 50 bis 150 mmol/l Puffersubstanz für pH 7,5 bis 8,5 enthält.

8. Reagenz nach einem der Ansprüche 4 bis 7, dadurch gekennzeichnet, daß es 0,1 bis 0,5 Gew.-% eines nichtionischen Detergenzes enthält.

9. Reagenz nach einem der Ansprüche 4 bis 8, dadurch gekennzeichnet, daß es Phosphatpuffer oder Trispuffer enthält.

10. Reagenz nach einem der Ansprüche 4 bis 9, dadurch gekennzeichnet, daß es als Tetrazoliumsalz 3-(4′, 5′-Dimethylthiazol-2-yl)-2,4-diphenyltetrazoliumbromid (MTT) oder 2,2′-di-(p-Nitrophenyl)-5,5′-diphenyl-3,3′-(3,3′-dimethoxy-4,4′-diphenylen)-ditetrazoliumchlorid (NBT) enthält.

## Claims

1. Process for the enzymatic determination of urea, characterised in that one reacts the urea with glyoxylate in the presence of (S)-ureidoglycolate synthetase to give (S)-ureidoglycolate and oxidises the latter with NAD(P)$^+$ and ureidoglycolate dehydrogenase to give carbamoyloxamate and measures NAD(P)H formed either directly or via a colour indicator system.

2. Process according to claim 1, characterised in that one reacts NAD(P)H with a tetrazolium salt in the presence of diaphorase to a formazan coloured material and measures the latter.

3. Process according to claim 1 or 2, characterised in that one uses ureidoglycolate dehydrogenase from Proteus rettgeri, DSM 2964.

4. Reagent for the enzymatic determination of urea, characterised in that it contains glyoxalate, NAD(P)$^+$, (S)-ureidoglycolate synthetase, ureidoglycolate dehydrogenase and buffer substance.

5. Reagent according to claim 4, characterised in that it additionally contains diaphorase and tetrazolium salt.

6. Reagent according to claim 5, characterised in that, in the form of an aqueous solution, it contains 0.1 to 10 mmol/l. glyoxylate, 1 to 20 mmol/l. NAD(P)$^+$, 1 to 30 U/ml. each of (S)-ureidoglycolate synthetase and ureidoglycolate dehydrogenase, 0.1 to 10 U/ml. diaphorase, 0.05 to 1 mmol/l. tetrazolium salt and 20 to 200 mmol/l. buffer substance pH 7 to 9.

7. Reagent according to claim 6, characterised in that it contains 0.5 to 5 mmol/l. glyoxylate, 5 to 10 mmol/l. NAD$^+$, 3 to 30 U/ml. each of (S)-ureidoglycolate synthetase and ureidoglycolate dehydrogenase, 0.5 to 5 U/ml. diaphorase and 0.1 to 0.5 mmol/l. tetrazolium salt and 50 to 150 mmol/l. buffer substance for pH 7.5 to 8.5.

8. Reagent according to one of claims 4 to 7, characterised in that it contains 0.1 to 0.5 wt.% of a nonionic detergent.

9. Reagent according to one of claims 4 to 8, characterised in that it contains phosphate buffer or tris buffer.

10. Reagent according to one of claims 4 to 9, characterised in that, as tetrazolium salt, it contains 3-(4′,5′-dimethylthiazol-2-yl)-2,4-diphenyltetrazolium bromide (MTT) or 2,2′-di-(p-nitrophenyl)-5,5′-diphenyl-3,3′-(3,3′-dimethoxy-4,4′-diphenylene)-ditetrazolium chloride (NBT).

## Revendications

1. Procédé pour la détermination enzymatique de l'urée, caractérisé en ce qu'on fait réagir l'urée avec du glyoxylate en présence de (S)-uréido-glycolate-synthétase pour donner le (S)-uréidoglycolate, et on oxyde ce dernier avec du NAD(P)$^+$ et de l'uréidoglycolate-déshydrogénase pour donner de l'oxamate de carbamoyle, et on mesure le NAD(P)H formé soit directement, soit par l'intermédiaire d'un système d'indicateur coloré.

2. Procédé selon la revendication 1, caractérisé en ce qu'on fait réagir le NAD(P)H avec un sel de tétrazolium en présence de diaphorase, pour donner un colorant formazan et on mesure ce dernier.

3. Procédé selon la revendication 1 ou 2, caractérisé en ce qu'on utilise de l'uréidoglycolate-déshydrogénase de Proteus rettgeri, DSM 2964.

4. Réactif pour la détermination enzymatique de l'urée, caractérisé en ce qu'il contient du glyoxylate, du NAD(P)$^+$, de la (S)-uréidoglycolate-synthétase, de l'uréidoglycolate-déshydrogénase et une substance tampon.

5. Réactif selon la revendication 4, caractérisé en ce qu'il contient en outre de la diaphorase et un sel de tétrazolium.

6. Réactif selon la revendication 5, caractérisé en ce qu'il contient sous forme de solution aqueuse 0,1 à 10 mmoles/l de glyoxylate, 1 à 20 mmoles/l de NAD(P)$^+$, 1 à 30 U/ml de (S)-uréido-glycolate-synthétase, 1 à 30 U/ml d'uréidoglycolate-déshydrogénase, 0,1 à 10 U/ml de diaphorase, 0,05 à 1 mmole/l de sel de tétrazolium et 20 à 200 mmoles/l de substance tampon pH 7 à 9.

7. Réactif selon la revendication 6, caractérisé en ce qu'il contient 0,5 à 5 mmoles/l de glyoxylate, 5 à 10 mmoles/l de NAD$^+$, 3 à 30 U/ml de S-uréidoglycolate-synthétase, 3 à 30 U/ml d'uréidoglycolate-déshydrogénase, 0,5 à 5 U/ml de diaphorase et 0,1 à 0,5 mmole/l de sel de tétrazolium et 50 à 150 mmoles/l de substance tampon pour un pH de 7,5 à 8,5.

8. Réactif selon l'une des revendications 4 à 7, caractérisé en ce qu'il contient 0,1 à 0,5 % en poids d'un détergent non-ionique.

9. Réactif selon l'une des revendications 4 à 8, caractérisé en ce qu'il contient du tampon phosphate ou du tampon Tris.

10. Réactif selon l'une des revendications 4 à 9, caractérisé en ce qu'il contient comme sel de tétrazolium du bromure de 3-(4',5'-diméthylthiazol-2-yl)-2,4-diphényltétrazolium (MTT) ou du chlorure de 2,2'-di-(p-nitrophényl)-5,5'-diphényl-3,3'-(3,3'-diméthoxy-4,4'-diphénylène)-ditétrazolium (NBT).

c Harnstoff i.d. Probe [mmol / l]

5,0   3,75   2,5   1,25   0,5

$\Delta E_{546\ nm}$

0,7   0,6   0,5   0,4   0,3   0,2   0,1